(19) Europäisches Patentamt
European Patent Office
Office européen des brevets

(11) **EP 4 606 393 A1**

(12) **EUROPEAN PATENT APPLICATION**

(43) Date of publication:
**27.08.2025 Bulletin 2025/35**

(21) Application number: **24159188.2**

(22) Date of filing: **22.02.2024**

(51) International Patent Classification (IPC):
**A61L 2/08** (2006.01)     **A61L 2/10** (2006.01)
**B26B 21/00** (2006.01)

(52) Cooperative Patent Classification (CPC):
**A61L 2/10; A61L 2/08; B26B 21/405;** A61L 2202/14

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Koninklijke Philips N.V.
5656 AG Eindhoven (NL)**

(72) Inventors:
• **GÖZÜTOK, Ahmet
5656 AG Eindhoven (NL)**
• **RAO, Ramachandra Ganesh
5656 AG Eindhoven (NL)**

(74) Representative: **Philips Intellectual Property &
Standards
High Tech Campus 52
5656 AG Eindhoven (NL)**

(54) **A SHAVING SYSTEM COMPRISING A SANITIZING DEVICE**

(57) Proposed are schemes, solutions, concepts, designs, methods and systems pertaining to sanitizing a shaving device having an internal member and an external member having hair entry openings that expose parts of the internal member. Specifically, during a sanitizing time period the shaving device is illuminated with sanitizing light. It is proposed to generate relative movement of the internal member and the external member within the sanitizing time period such that different parts of the internal member are exposed to the sanitizing light via the hair entry openings during respective different parts of the sanitizing time period. Accordingly, the internal member may be more thoroughly sanitized.

FIG. 1

**Description**

FIELD OF THE INVENTION

The present invention relates to a shaving system comprising a shaving device, a sanitizing device and a processing unit for controlling a light source of the sanitizing device to illuminate parts of the shaving device with sanitizing light.

The present invention further relates to a sanitizing device configured to sanitize a shaving device and having a processing unit for controlling a light source of the sanitizing device to illuminate parts of the shaving device with sanitizing light.

The present invention further relates to a method for sanitizing a shaving device comprising controlling a light source to illuminate parts of the shaving device with sanitizing light.

BACKGROUND OF THE INVENTION

It is known that sanitizing light (e.g., ultraviolet light) is useful for improving the hygiene of personal care devices. Adequate exposure to sanitizing light may deactivate common bacteria on personal care devices, reducing the spread of such bacteria. This may be particularly useful for shaving devices, as shaving devices contact skin of the user, and may incidentally produce cuts on the skin of the user that must remain sterile to reduce risk of infection.

The use of sanitizing light may be advantageous over, for example, a liquid-based cleaner, as no residue is left behind. Furthermore, use of liquid-based cleaners may risk damaging electronics of the shaving device, or degrading materials of the shaving device. In contrast, sanitizing light causes little to no deterioration of the shaving device.

Nevertheless, the efficacy of a light-based sanitizing system relies on the adequate exposure of a surface to sanitizing light.

KR20110126002A discloses a sanitizing device which is integrally formed with a protective cap for a shaving unit of an electric shaver, wherein the shaving unit comprises an internal hair-cutting member and an external hair-cutting member covering the internal member and including hair entry openings which expose parts of the internal hair-cutting member. The known sanitizing device comprises UV LEDs which are arranged to illuminate the shaving unit when the protective cap is placed on the shaver to cover the shaving unit. Although in this way the external hair-cutting member is substantially entirely exposed to the UV light, parts of the internal hair-cutting member will be covered by the external hair-cutting member and, as a result, will not be exposed to the UV light. As a result, the internal hair-cutting member will not be adequately exposed to the UV light and bacteria will not be adequately deactivated. Since during shaving parts of the internal hair-cutting member might contact the skin, there will still be a risk of skin infection.

SUMMARY OF THE INVENTION

The invention is defined by the claims.

An object of the present invention is to provide a shaving system comprising a shaving device and a sanitizing device for sanitizing the shaving device by sanitizing light, by means of which the disadvantage of the known sanitizing device described herein before is at least mitigated. An object of the present invention therefore is to provide such a shaving system with an improved sanitizing efficacy.

According to examples in accordance with an aspect of the invention, there is provided a shaving system comprising a shaving device, a sanitizing device, and a processing unit, wherein:

the shaving device comprises an internal member, an external member covering the internal member and including hair entry openings exposing parts of the internal member, and an actuator configured to generate relative movement of the internal member and the external member, wherein the external member and the internal member are configured to cut hair passing through the hair entry openings during relative movement of the internal member and the external member;

the sanitizing device comprises a light source configured to illuminate the external member and the exposed parts of the internal member with sanitizing light in a predefined sanitizing position of the shaving device relative to the sanitizing device; and

the processing unit is configured to:

control the light source to illuminate the external member and the exposed parts of the internal member with sanitizing light within a sanitizing time period; and
control the actuator to generate relative movement of the internal member and the external member within the sanitizing time period such that different parts of the internal member are exposed to the sanitizing light via the hair entry openings during respective different parts of the sanitizing time period.

Proposed concepts thus aim to provide schemes, solutions, concepts, design methods and systems pertaining to a shaving system that is adapted to facilitate light-based sanitizing of the shaving system's shaving device.

The proposed shaving system facilitates the sanitizing (e.g. cleansing and/or sterilizing) of a shaving device using light (such as UV light, for example). Specifically, during a sanitizing time period, the shaving device is illuminated with sanitizing light. However, an external member and an internal member of the shaving device configured to cut hair passing hair entry opening of the external member are positioned such that the external member covers the internal member. As a result, only parts of the internal member are exposed to the sanitizing light. It is proposed to generate relative movement of the internal member and the external member within the sanitizing time period such that different parts of the internal member are exposed to the sanitizing light via the hair entry openings during respective different parts of the sanitizing time period. Accordingly, by generating relative movement of the internal member and the external member within the sanitizing time period, parts of the internal member which are covered by the external member at particular moments during the sanitizing time period may be exposed to the sanitizing light via the hair entry openings at other moments during the sanitizing time period. As a result, the internal member may be sanitized more completely and more thoroughly.

Typically, in conventional approaches such as described in KR20110126002A, the parts of the internal member that are covered by the external member would not be sanitized, as the sanitizing light may not directly illuminate these parts. However, it is proposed that by controlling the actuator of the shaving device to move the internal member and the external member relative to each other, different parts of the internal member may be successively exposed to the sanitizing light via the hair entry openings of the external member. Performing both the movement and the illumination within the sanitizing time period implies that parts of the internal member, that would otherwise remain unilluminated, will be exposed to the sanitizing light during the sanitizing time period.

In other words, if the light source were controlled in a conventional manner to illuminate the external member and the exposed parts of the internal member with sanitizing light, with no relative movement between the internal and external members, then only the exposed parts of the internal member will be sanitized. As a result, any parts of the internal member covered by the external member, in particular by parts of the external member situated between the hair entry openings, would not be sanitized. However, by employing the proposed concept of controlling the actuator to generate relative movement of the internal member and the external member within the sanitizing time period, different parts of the internal member are exposed to the sanitizing light via the hair entry openings. Accordingly, these different parts, which otherwise would not be sanitized, will be exposed to sanitizing light in proposed embodiments.

Ultimately, an improved shaving system may be provided by the proposed concept(s).

In some embodiments, the shaving system may further comprise a positioning arrangement configured to position the shaving device in the predefined sanitizing position relative to the sanitizing device.

The positioning arrangement therefore co-locates the light source and the shaving device such that the external member and the exposed parts of the internal member are illuminated with sanitizing light when the light source is active. This may ensure proper illumination of the external member and the exposed parts of the internal member by the light source, and thereby ensure adequate sanitization of the shaving device.

Furthermore, the sanitizing device may further comprise a casing configured to receive at least a shaving unit of the shaving device. The casing may include the positioning arrangement and the shaving unit may include the internal member and the external member.

The casing (e.g., a housing) may thus hold part of the shaving device, the shaving unit, so that the external member and the exposed parts of the internal member are illuminated with sanitizing light when the light source is active.

In specific embodiments, the processing unit may be part of the sanitizing device.

That is, the processing unit may be integrated into the same structure as the light source. This may provide a self-contained case that can be used to sanitize the shaving device when the shaving device is provided in the predefine sanitizing position relative to the sanitizing device.

The processing unit may be configured to control the actuator via data communication with a control unit of the shaving device enabled in the sanitizing position of the shaving device.

In other words, the processing unit controls the actuator of the shaving device to generate the relative movement when the shaving device is in the sanitizing position relative to the sanitizing device. This is facilitated by data communication with a control unit of the shaving device that can, in turn, cause the actuator to generate the relative movement. For example, the processing unit may simply prompt the control unit to cause the actuator to generate relative movement, or may provide a control signal to the control unit, which then uses said control signal to control movement of the actuator.

In some embodiments, the processing unit may be configured to control the actuator to continuously move the internal member relative to the external member throughout the sanitizing time period.

Alternatively, the processing unit may be configured to control the actuator to move the internal member relative to the external member during one or more discrete parts of the sanitizing time period.

Thus, the actuator either affects continual relative movement from start to end of the sanitizing time period, or the actuator may affect movement during different parts of the sanitizing time period, with different or no movement in between these parts of the sanitizing time period. In any case, this relative movement during the sanitizing time period means that different parts of the internal member are exposed via the hair entry openings of the external member at different moments during the sanitizing time period.

Furthermore, the processing unit may be configured to control the actuator to move the internal member relative to the external member within the sanitizing time period at a predefined movement speed.

The predefined movement speed may be a particular movement speed defined for improved exposure of different parts of the internal member to the sanitizing light.

More specifically, the processing unit may be further configured to control the actuator to move the internal member relative to the external member at an operational movement speed for cutting hair. The predefined movement speed in this case may be less than the operational movement speed.

By setting two distinct speeds of the relative movement, an appropriate speed of relative movement may be affected when the shaving device is operating in different circumstances. That is, a fast speed may be required for effective hair cutting, whilst a slower speed may be acquired for effective sanitization of the shaving device.

In some embodiments, the processing unit may be configured to control the light source to continuously illuminate the external member and the exposed parts of the internal member with the sanitizing light throughout the sanitizing time period.

Alternatively, the processing unit may be configured to control the light source to illuminate the external member and the exposed parts of the internal member with sanitizing light at a predefined pulse frequency within the sanitizing time period.

Similarly to the movement affected by the actuator, the light source may continually illuminate the shaving device during the sanitizing time period. Alternatively, the light source may be switched on or off at different points during the sanitizing time period. More specifically, the sanitizing light may be provided in pulses. In any case, at different moments of the sanitizing time period the sanitizing light will illuminate different parts of the internal member.

In particular, the processing unit may be configured to control the light source to illuminate the external member and the exposed parts of the internal member with the sanitizing light at a predefined pulse frequency within the sanitizing time period. In this case, the predefined movement speed and the predefined pulse frequency are adapted such that different parts of the internal member are exposed to the sanitizing light via the hair entry openings during respective different parts of the sanitizing time period.

That is, it is desirable to predefine the movement speed of the internal member and the pulse frequency of the light source in such a way that different parts of the internal member are exposed through the hair openings during successive light pulses of the light source. For example, it would be disadvantageous if the frequency of oscillatory relative movement matched that of the light pulse frequency, as the same part of the internal member would be exposed whenever the light is turned on. Therefore, in the case that pulsed light is provided by the light source, the frequency of said pulsed light and the movement speed should be adapted to avoid this.

The processing unit in some embodiments may be configured to control the light source and the actuator to illuminate the external member and the exposed parts of the internal member with the sanitizing light while the actuator moves the internal member relative to the external member within the sanitizing time period.

Accordingly, the processing unit may control the light source and the actuator to simultaneously generate the sanitizing light and move the internal member, so that different parts of the internal member are exposed continually as the light illuminates the shaving device.

In some cases, the sanitizing light comprises ultraviolet light.

Ultraviolet light may be particularly effective for sanitizing a surface. Indeed, it is known that ultraviolet light of sufficient intensity can be used to deactivate common bacteria. Nevertheless, other wavelengths of light, such as blue light, may also be present in the sanitizing light, and may also have sanitizing properties. The sanitizing effects of light of different wavelengths and intensities are well understood by the skilled person.

According to another aspect of the invention, there is provided a sanitizing device configured to sanitize a shaving device, wherein the shaving device comprises:

a shaving unit having an internal member and an external member covering the internal member and including hair entry openings exposing parts of the internal member; and
an actuator configured to generate relative movement of the internal member and the external member, wherein the external member and the internal member are configured to cut hair passing through the hair entry openings during relative movement of the internal member and the external member,
and wherein the sanitizing device comprises:

a casing configured to receive at least the shaving unit of the shaving device and including a positioning arrangement configured to position the shaving device in a predefined sanitizing position relative to the sanitizing device;

a light source configured to illuminate the external member and the exposed parts of the internal member with sanitizing light in the predefined sanitizing position of the shaving device; and

a processing unit configured to, in the predefined sanitizing position of the shaving unit:

control the light source to illuminate the external member and the exposed parts of the internal member with sanitizing light within a sanitizing time period; and

control the actuator to generate relative movement of the internal member and the external member within the sanitizing time period such that different parts of the internal member are exposed to the sanitizing light via the hair entry openings during respective different parts of the sanitizing time period.

According to still another aspect of the invention, there is provided a method for sanitizing a shaving device comprising an internal member, an external member covering the internal member and including hair entry openings exposing parts of the internal member, and an actuator configured to generate relative movement of the internal member and the external member, wherein the external member and the internal member are configured to cut hair passing through the hair entry openings during relative movement of the internal member and the external member, the method comprising:

controlling a light source to illuminate the external member and the exposed parts of the internal member with sanitizing light in a predefined sanitizing position of the shaving device relative to the sanitizing device within a sanitizing time period; and

controlling the actuator to generate relative movement of the internal member and the external member within the sanitizing time period such that different parts of the internal member are exposed to the sanitizing light via the hair entry openings during respective different parts of the sanitizing time period.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 is a simplified block diagram of a shaving system according to an embodiment;
Fig. 2 is a simplified block diagram of a shaving system according to another embodiment;
Fig. 3 shows in detail an example external member and internal member of a shaving device of the shaving systems of Figs. 1 and 2;
Fig. 4 presents another example external member of a shaving device of the shaving systems of Figs. 1 and 2, and an abstracted view of said external member;
Fig. 5 presents a flow diagram of a method for sanitizing a shaving device according to an example embodiment; and
Fig. 6 provides a simplified block diagram of a computer within which one or more parts of an embodiment may be employed.

DETAILED DESCRIPTION OF THE EMBODIMENTS

The invention will be described with reference to the Figures.
It should be understood that the Figures are merely schematic and are not drawn to scale. It should also be understood that the same reference numerals are used throughout the Figures to indicate the same or similar parts.
It should also be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. The mere fact that certain measures are recited in mutually different dependent claims does not indicate that a combination of these measures cannot be used to advantage.
Proposed are schemes, solutions, concepts, designs, methods and systems pertaining to sanitizing a shaving device. Specifically, during a sanitizing time period the shaving device is illuminated with sanitizing light. An external member and an internal member of the shaving device, which are configured to cut hair passing hair entry openings of the external member, are arranged such that the external member covers the internal member to a certain extent. As a result, without any further measures, only some parts of the internal member are exposed to the sanitizing light and other parts of the internal member are not exposed to the sanitizing light. To achieve exposure of more parts of the internal member to the sanitizing light, in particular those parts of the internal member that may contact the skin during a shaving operation, it is proposed to generate relative movement of the internal member and the external member within the sanitizing time period such that different parts of the internal member are exposed to the sanitizing light via the hair entry openings during respective different parts of the sanitizing time period. Accordingly, the internal member may be more thoroughly sanitized.

The disclosed invention provides a means for improving the efficacy of a light-based sanitization process of a shaving device. When light is used to sanitize a surface of the shaving device with holes, the parts of the shaving device shadowed by the surface are not exposed to the light. Hence, these shadowed parts cannot be sanitized by the light. Accordingly, the invention proposes to affect movement between an external member with hair entry openings and an internal member covered by the external member, such that the parts of the internal member shadowed by the external member at a certain moment during the exposure may be exposed to the light at another moment during the exposure. Indeed, a shaving device usually has an actuator capable of affecting relative movement between the external member and the internal member in order to perform hair cutting, and therefore this solution may be implemented in shaving devices without substantially modifying existing shaving devices.

More particularly, there is provided a shaving system having a sanitizing device designed to radiate, by means of a light source, a sanitizing light (e.g., ultraviolet light) towards the outer shaving surface of a shaving unit to inactivate the common bacteria on the surface touching the skin.

The shaving unit comprises an external member and an internal member beneath the external member, i.e., covered by the external member. The external member and internal member are configured to cut hair passing through hair entry openings of the external member as the external member and internal member move relative to each other (e.g., as the internal member, and/or the external member moves/rotates/oscillates). For example, the internal member may be a blade configured to rotate beneath the external member, thus cutting hair passing through the hair entry openings when the external member is in contact with skin of a user. Alternatively, the external member may be a blade, and the internal member may oscillate beneath the blade, thus manipulating hair to enter the hair entry openings, and forcing the hair against the blade. Several types of shaving units comprising an external member with hair entry openings and an internal member configured to cut hair by relative movement are readily apparent to the skilled person.

In any case, since the sanitizing light is partially blocked by the external member, the internal member cannot be fully exposed to the sanitizing light. The only parts of the internal member exposed to the sanitizing light are those parts which are exposed via the hair entry openings. Thus, it is proposed, during a sanitizing time period, to move the internal member relative to the external member and, thereby, to effectively move different parts of the internal member, that would otherwise remain unexposed, into positions wherein these parts are exposed via the hair entry openings.

It is an aim of the present invention to suitably sanitize all surfaces of a shaving device that may touch skin of the user during shaving, preferably with more than 99% efficacy. However, without any further measures, the parts of the internal member, which typically touch the skin via the hair entry openings during shaving, cannot all be sanitized with 99% efficacy since the external member partially blocks the light. There are some openings on the external member, i.e. the hair entry openings, that enable light to penetrate through the external member to the internal member, but some parts of the internal member cannot be reached and cannot be sanitized. Since the internal member often comprises blades or cutting elements to cut the hairs entering through the hair entry openings, and these blades or cutting elements may cause micro cuts on the skin, sanitizing of all parts of the internal member that may contact the skin during shaving is key to ensuring efficacy of the sanitizing process.

Accordingly, disclosed embodiments control an actuator to move (e.g., rotate, oscillate, etc.) the internal member relative to the external member during a sanitizing time period. This movement may occur at a predetermined speed, such that the internal member is adequately exposed to the sanitizing light and therefore adequately sanitized.

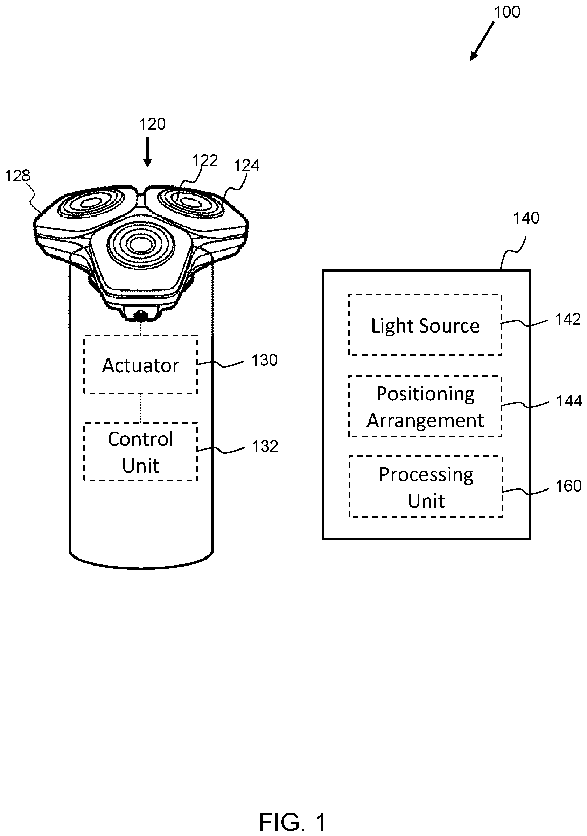

Fig. 1 presents a simplified block diagram of a shaving system 100 according to an embodiment of the invention. In particular, the shaving system 100 comprises a shaving device 120, a sanitizing device 140 according to an embodiment of the invention, and a processing unit 160. The shaving device 120 comprises a shaving unit 128 including an internal member 122 (not visible) and an external member 124 covering the internal member 122, an actuator 130 for affecting movement of the internal and/or external member 124, and also comprises a control unit 132 for controlling the actuator 130. The sanitizing device 140 comprises a light source 142, and a positioning arrangement 144. In the presented embodiment of the shaving system 100, the sanitizing device 140 comprises a processing unit 160.

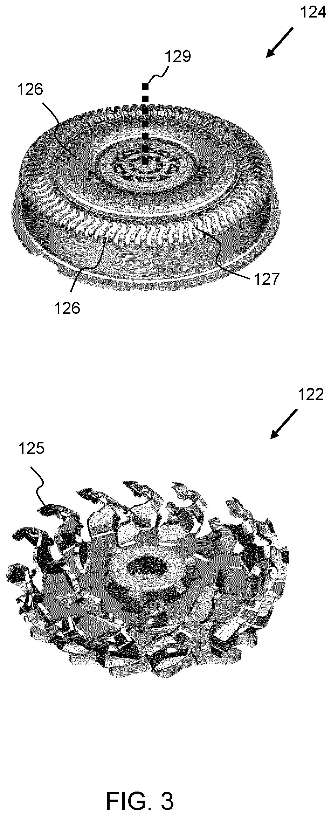

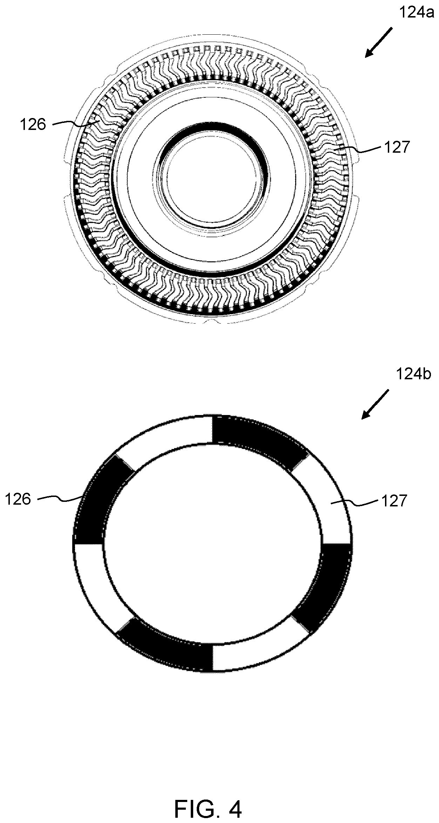

More specifically, the shaving device 120 comprises an internal member 122 (e.g., a covered cutting blade), an external member 124 (e.g., a cap, guard, or covering) covering the internal member 122. The external member 124 comprises one or more hair entry openings 126, shown in Figs. 3 and 4, configured to receive hair of the user when the external member 124 is in contact or in close proximity with skin of the user. One or more parts of the internal member 122 are exposed by the hair entry openings 126. The internal member 122 and the external member 124 are movable relative to one another. That is, at least one of the internal member 122 and external member 124 is movable to generate relative movement between the internal member 122 and the external member 124. The external member 124 and the internal member 122 are configured to cut hair passing through the hair entry openings 126 during relative movement of the internal member 122 and the external member 124. When hair is in one of the hair entry openings 126, and the internal member 122 and/or the external member 124 move, the internal member 122 and the external member 124 affect cutting of the hair. This may be, for example, because at least one of the internal member 122 or the external member 124 has a sharp cutting edge.

By way of specific example, the external member 124 may be a cap having a plurality of slots 126 for receiving hair, and a plurality of lamellas 127 between the slots 126, as shown in Figs. 3 and 4. The slots 126 and lamellas 127 may be arranged alternately within an annular shaving track of the external member 124, as is well known to the skilled person. An internal member 122 is provided, and may be shaped to cut hair entering through the slots 126 as it rotates beneath the cap. A surface of each lamella 127 facing a respective slot 126 may have a sharp edge so as to cut hair passing through in cooperation with a cutting edge of the internal member 122. Furthermore, there may be multiple shaving tracks with slots and lamellas to increase the effective shaving area.

Of course, this is by way of example only. The external member 124 may be a cover with hair entry openings, and the internal member 122 may be a blade configured to move back and forth over the gaps, thereby cutting hair entering through the hair entry openings. Other types of shaving units may be applied and would be readily appreciated by the skilled person.

The shaving device 120 also comprises an actuator 130 configured to generate the relative movement of the internal member 122 and the external member 124. That is, the actuator 130 may move one (or both) of the internal member 122 and the external member 124 so that the internal member 122 and the external member 124 move with respect to each other. Said movement may be an oscillatory movement, and in particular a rotational movement, but embodiments are not restricted hereto.

The actuator 130 may be capable of affecting the relative movement at different speeds depending on the operation of the shaving device 120. For example, the actuator 130 may generate relative movement at a predefined movement speed suitable for sanitizing the internal member 122. In addition, the actuator 130 may also (in a different mode of operation) generate relative movement at a predefined operational movement speed suitable for cutting hair entering the hair entry openings 126. The predefined movement speed for sanitizing (i.e., the sanitizing movement speed) may be lower than that of the predefined operational movement speed for hair cutting (i.e., the cutting/shaving movement speed). Indeed, the predefined movement speed for sanitizing may be an order of magnitude lower (or more than an order of magnitude lower) than that of the predefined operational movement speed for hair cutting. There may also be multiple different predefined movement speeds available for selection based on the type of sanitization required and/or user preferences.

The light source 142 is configured to illuminate the external member 124 and the exposed parts of the internal member 122 with sanitizing light when the shaving device 120 is placed/provided/held in a predefined sanitizing position relative to the sanitizing device 140. Thus, when the shaving device 120 is provided in the sanitizing position, the light source 142 will illuminate the shaving device 120, i.e., at least the parts of the shaving device 120 that need to be sanitized including the external member 124 and the internal member 122.

The positioning arrangement 144 may be configured to position/hold the shaving device 120 in the predefined sanitizing position relative to the sanitizing device 140. For example, the positioning arrangement 144 may be a stand or holder for the shaving device 120. Furthermore, the sanitizing device 140 may comprise a casing configured to receive at least the shaving unit of the shaving device 120 (and potentially the whole shaving device 120). The casing may include the positioning arrangement 144. Accordingly, the sanitizing device 140 may be provided in one integrated structure for receiving and sanitizing the shaving device 120.

Thus, for example, the sanitizing device 140 may be provided as an openable and closeable storage case for the shaving device 120, wherein the light source 142 is arranged in an internal space of the storage case such that the shaving unit 128 is exposed to the light of the light source 142 when the shaving device 120 is arranged in a predefined stored position in the storage case and the storage case is closed. In such an execution, the positioning arrangement 144 may be a cavity within the storage case for holding the shaving device 120. Such storage cases for shaving devices having an integrated sanitizing light source are well known to the skilled person. Examples of such storage cases with an integrated sanitizing device are disclosed by KR200350028Y1 and CN214963194U. Alternatively, the sanitizing device 140 may be provided as a protective cap for the shaving unit 128 of the shaving device 120. In such an execution, the positioning arrangement 144 may be a circumferential edge portion of the protective cap arranged to position the cap in a defined position on the shaving device. An example of such a protective cap with an integrated sanitizing device is disclosed by KR20110126002A, as already mentioned herein before. Because all such executions are well known to the skilled person, the positioning arrangement 144 is only schematically indicated in Fig. 1 and no particular details are shown in relation to the structural execution thereof.

In particular embodiments, the light source 142 may generate ultraviolet light, and illuminate the shaving device 120 with ultraviolet light. Of course, other suitable wavelength ranges of light may be used for sanitizing purposes.

The processing unit 160 is configured to control the light source 142 to illuminate the external member 124 and the exposed parts of the internal member 122 with sanitizing light within a sanitizing time period. That is, during at least a part of the sanitizing time period, the processing unit 160 causes the light source 142 to illuminate the shaving device 120. The control may be as simple as causing the light source 142 to turn on, or may comprise controlling the light source 142 based on various light source 142 parameter values (e.g., describing an intensity, direction, frequency etc. of the light).

In some embodiments, the processing unit 160 may be configured to control the light source 142 to continuously illuminate the external member 124 and the exposed parts of the internal member 122 with the sanitizing light throughout the sanitizing time period. That is, the sanitizing light may be generated for the whole of the sanitizing time period.

The sanitizing time period is a length of time for sanitizing of the shaving device 120. The sanitizing time period may begin, for example, when the shaving device 120 is provided in the defined sanitizing position. Alternatively, the sanitizing time period may begin when a user provides an input to begin the sanitizing process. The sanitizing time period may be a predetermined length of time, may be determined by the user, or may continue until the shaving device 120 has been satisfactorily sanitized.

The processing unit 160 is further configured to control the actuator 130 to generate relative movement of the internal member 122 and the external member 124 within the sanitizing time period such that different parts of the internal member 122 are exposed to the sanitizing light via the hair entry openings 126 during respective different parts of the sanitizing time period.

The processing unit 160 causes the actuator 130 to generate the relative movement such that the exposed part of the internal member 122 is changed during the sanitizing time period in a manner such that the sanitizing light is provided to different parts of the internal member 122 at different times within the sanitizing time period.

In some embodiments, the processing unit 160 may be configured to control the actuator 130 to continuously move the internal member 122 relative to the external member 124 throughout the sanitizing time period. This means that the part of the internal member 122 that is actually exposed to the sanitizing light continually changes during the sanitizing time period.

The processing unit 160 in some embodiments may therefore be configured to control the light source 142 and the actuator 130 to illuminate the external member 124 and the exposed parts of the internal member 122 with the sanitizing light while the actuator 130 moves the internal member 122 relative to the external member 124 within the sanitizing time period.

The processing unit 160 thus controls both the illumination of the shaving device 120 and the relative movement of the internal member 122 and the external member 124 of the shaving device 120 at the same time. Of course, in alternative embodiments, the actuator 130 may be controlled to generate relative movement at discrete points in time, and the light source 142 may be controlled to illuminate the shaving device 120 only between these points in time.

Indeed, the processing unit 160 may be configured to control the actuator 130 to move the internal member 122 relative to the external member 124 during one or more discrete parts of the sanitizing time period.

For example, the processing unit 160 may first control the light source 142 to illuminate the shaving device 120 (illuminating a first part of the internal member 122), then may control the actuator 130 to generate relative movement of the internal member 122 and the external member 124, and then may control the light source 142 to illuminate the shaving device 120 again (illuminating a second, different, part of the internal member 122). This process may be repeated until all parts of the internal member, which may be exposed by the hair openings of the external member 124, have been illuminated with the sanitizing light.

In some cases, the light source 142 may be configured to generate pulses of sanitizing light. The pulses of sanitizing light may thus be generated at a predefined pulse frequency. In these cases, it may be important to carefully adapt the predefined movement speed to ensure that, indeed, distinct parts of the internal member 122 are exposed to the sanitizing light via the hair entry openings 126 during respective different parts of the sanitizing time period.

In particular, the processing unit 160 may be configured to control the light source 142 to illuminate the external member 124 and the exposed parts of the internal member 122 with the sanitizing light at a predefined pulse frequency within the sanitizing time period. In this case, the predefined movement speed and the predefined pulse frequency are adapted such that different parts of the internal member 122 are exposed to the sanitizing light via the hair entry openings 126 during respective different parts of the sanitizing time period.

For example, if the predefined movement speed would be such that the same parts of the internal member 122 is exposed every 0.5 seconds, and the predefined pulse frequency would be 2Hz, then said same parts of the internal member 122 would be exposed to each pulse of the sanitizing light and other parts of the internal member 122 would not be exposed to the sanitizing light. This will be explained in more detail below in reference to Fig. 4.

Furthermore, in some embodiments, the processing unit 160 may be configured to receive a user input describing a desired pulse frequency of the light source 142 or a desired movement speed of the relative movement of the internal member 122 and the external member 124. In embodiments wherein the user input describes the desired pulse frequency, e.g., 200 Hz, the processing unit 160 may determine the movement speed in dependence on the desired pulse frequency. In embodiments wherein the user input describes the desired movement speed, e.g., a rotational movement speed of 40 rpm of the internal member 122, the processing unit 160 may determine the pulse frequency in dependence on the desired movement speed. The user may provide this input via an application on a mobile device, or via a user interface integrated with the shaving device 120 or the sanitizing device 140.

The processing unit 160 may be configured to control the actuator 130 via data communication with the control unit 132 of the shaving device 120 enabled in the sanitizing position of the shaving device 120.

Said data communication may be wireless (e.g., Bluetooth, Zigbee or Wi-Fi), or may be enabled by a wired connection enabled in the sanitizing position. In the example of data communication via a wired connection, said wired connection may also be used to charge a rechargeable battery of the shaving device 120 when the shaving device 120 is in the sanitizing position. Essentially, the data communication enables the processing unit 160 to control either or both of the actuator 130 and the light source 142 (via respective controllers, if applicable).

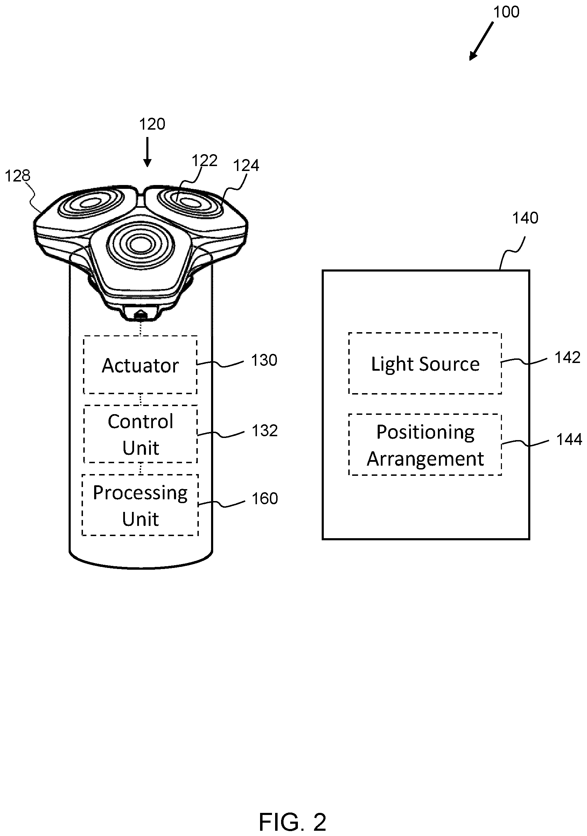

Fig. 2 presents a simplified block diagram of a shaving system 100 according to another embodiment of the invention. Components of the shaving system 100 shown in Fig. 2 may be substantially the same as those described in reference to the embodiment shown in Fig. 1. However, in contrast to the embodiment of Fig. 1, the processing unit 160 of the embodiment of Fig. 2 is provided as part of the shaving device 120 rather than the sanitizing device 140.

In the embodiment shown in Fig. 2, the processing unit 160 may be configured to control the light source 142 of the sanitizing device 140 via data communication with a control unit of the sanitizing device 140 enabled when the shaving device 120 is in the sanitizing position.

While not depicted in the figures, the processing unit 160 may alternatively be provided as part of a separate or remote device. In this case, the processing unit 160 may be configured to control both the actuator 130 of the shaving device 120 and the light source 142 of the sanitizing device 140 via data communication with respective control units of the shaving device 120 and the sanitizing device 140. For example, the processing unit 160 may be provided as part of a mobile device such as a smart phone.

Fig. 3 presents a detailed view of an example external member 124 and internal member 122 of the shaving device 120.

As can be seen, the external member 124 comprises an annular track of hair entry openings 126 separated by lamellas 127. As also depicted, the external member 122 may comprise multiple annular tracks of hair entry openings 126 arranged concentrically. The presented external member 124 is provided in the form of a cap that can be provided over the internal member 122.

The internal member 122 comprises an annular array of cutting elements 125 which each have a sharp cutting edge arranged to cooperate with cutting edges provided on the lamellas 127 of the external member 122. In particular the tip portions of said cutting elements 125 may contact the skin during shaving and, accordingly, should be exposed to the sanitizing light via the hair entry openings 126 during the sanitizing process.

The internal member 122 and external member 124 are configured so as to work together to cut hair passing through the hair entry openings 126 of the external member 124. Accordingly, when the internal member 122 is positioned beneath the external member 124, and the internal member 122 and the external member 124 are moved relative to one another, hair passing through the hair entry openings 126 may be cut by interaction of the cutting edges of the lamellas 127 and the cutting edges of the cutting elements 125. In particular the internal member 122 may be rotated relative to the external member 124 about a central axis 129 of the external member 124.

As will be appreciated, other configurations of the internal member 122 and the external member 124 are possible, and the configuration presented in Fig. 3 is meant by way of illustrative example only.

Fig. 4 presents a schematic view of an external member 124a of a shaving device according to an aspect of the invention, and an abstracted view of the external member 124b.

As shown, the external member 124a (e.g., a cap, guard, or cover) comprises a plurality of hair entry openings 126. The hair entry openings 126 are separated by lamellas 127 of the external member 124a. The lamellas 127 may be shaped such that they contribute to cutting hair in one of the hair entry openings 126 when the hair is forced toward the lamella (e.g., by the relative movement of the internal member 122 and external member 124).

In this case, the openings 126 are arranged circumferentially. Accordingly, the relative movement between the external member 124 and the internal member 122 may be a rotational movement. That is, the external member 124 and the internal member 122 may be coaxial, with one or both of the external member 124 and internal member 122 rotated by the actuator 130. Nevertheless, in other examples of shaving units the movement may equally be a linear or other kind of movement, and the arrangement of the hair entry openings 126 may be adapted to reflect this.

When the internal member 122 is covered by the presented external member 124a, parts of the internal member 122 will be shadowed by the external member 124a. Specifically, parts of the internal member 122 covered by the lamellas 127 of the external member 124a will not be exposed to the sanitizing light when the sanitizing light is radiated toward the external member 124a. As the internal member 122 is typically placed very close to the external member 124a (in order to facilitate a joint cutting action), a light-scattering effect under the external member 124a may be insignificant and largely ignored. Hence, the parts of the internal member 122 covered by the lamellas 127 of the external member 124a between the hair entry openings 126 cannot be sanitized by the sanitizing light.

When relative movement is generated at a predefined movement speed and there is continuous sanitizing light generation, the parts of the internal member 122, which are beneath the lamellas 127 at a certain point in time, will be exposed to the sanitizing light at a next point in time. When relative movement is generated at a predefined movement speed and the sanitizing light is discrete (i.e., non-continuous), then the predefined speed of the relative movement must be adapted to avoid continuously shadowing the same parts of the internal member 122.

For example, the predefined speed of the relative rotational movement between the internal member 122 and the external member 124a will depend upon the number of lamellae 127, the surface area of the lamellae 127, the area of the hair entry openings 126, and the pulse frequency of the sanitizing light.

A simplified example is shown by the abstraction of the external member 124b in Fig. 4. As shown, there are four lamellae 127, and four hair entry openings 126 each having the same area. An adequate predefined relative movement speed (in rotations per minute) may be expressed as follows:

$$Rotation\ Speed = f_{exposure} / \left\{ \eta \times \frac{S_{lamella} + S_{opening}}{S_{lamella}} \right\} \qquad [1]$$

where $\eta$ is the number of lamellae, $S_{lamella}$ is the surface area of the lamellae, $S_{opening}$ is the area of the hair entry openings, and $f_{exposure}$ is the pulse frequency of sanitizing light. For the depicted example, $\eta = 4$, $\frac{S_{lamella} + S_{opening}}{S_{lamella}} = 2$, and $f_{exposure} = 200\text{Hz}$. In this case, the rotational speed may be 25 rpm. Of course, the rotation speed will vary with geometry of the external member 124 and the internal member 122, and therefore this calculation represents a simplified example only. Nevertheless, the same principles will apply to determine an adequate speed of relative movement to avoid shadowed areas of the internal member 122. Of course, in the alternative example of a given rotational speed, the pulse frequency of the sanitizing light may be determined in order to avoid the shadowed areas.

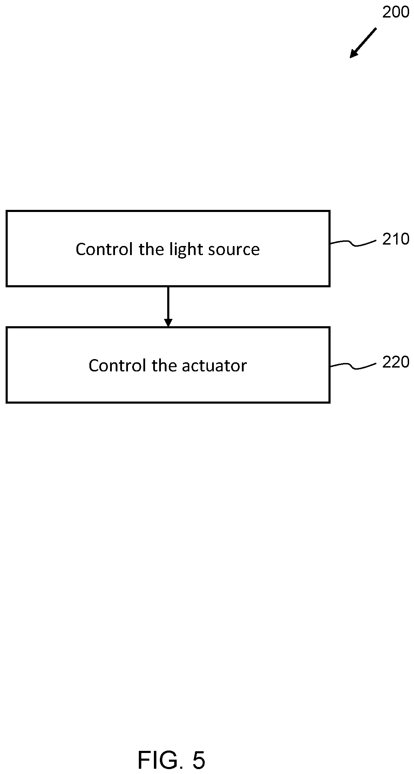

Fig. 5 presents a flow diagram of a method for sanitizing a shaving device. As described above, the shaving device comprises an internal member, an external member covering the internal member and including hair entry openings exposing parts of the internal member, and an actuator configured to generate relative movement of the internal member and the external member. The external member and the internal member are configured to cut hair passing through the hair entry openings during relative movement of the internal member and the external member.

In step 210, the light source is controlled to illuminate the external member and the exposed parts of the internal member with sanitizing light in a predefined sanitizing position of the shaving device relative to the sanitizing device within a sanitizing time period.

Similarly to the above, the light source may be controlled to continually illuminate the shaving device throughout the sanitizing time period. Of course, the light source may alternatively be controlled to illuminate the shaving device at discrete time periods within the sanitizing time period.

In step 220, the actuator is controlled to generate relative movement of the internal member and the external member within the sanitizing time period. This movement is generated such that different parts of the internal member are exposed to the sanitizing light via the hair entry openings during respective different parts of the sanitizing time period.

As with the light source, the actuator may be controlled to continually generate the relative movement throughout the sanitizing time period. Alternatively, the actuator may be controlled to generate the relative movement at discrete time periods within the sanitizing time period.

Accordingly, parts of the internal member that would otherwise be shadowed by the external member throughout the sanitizing period may be illuminated. Thus, a larger portion of the internal member may be sanitized by the proposed method.

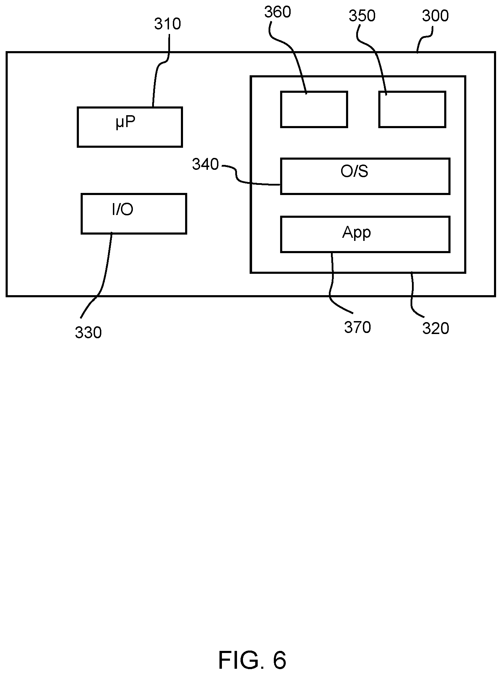

Fig. 6 illustrates an example of a computer 300 within which one or more parts of an embodiment may be employed. Various operations discussed above may utilize the capabilities of the computer 300. For example, one or more parts of a system for controlling a shaving device may be incorporated in any element, module, application, and/or component discussed herein. In this regard, it is to be understood that system functional blocks can run on a single computer or may be distributed over several computers and locations (e.g. connected via internet), such as a cloud-based computing infrastructure.

The computer 300 includes, but is not limited to, PCs, workstations, laptops, PDAs, palm devices, servers, storages, microcontroller units, integrated processors, AI-accelerators, and the like. Generally, in terms of hardware architecture, the computer 300 may include one or more processors 310, memory 320, and one or more I/O devices 330 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 310 is a hardware device for executing software that can be stored in the memory 320. The processor 310 can be virtually any custom made or commercially available processor, a central processing unit (CPU), a digital signal processor (DSP), a tensor processing unit (TSP) specifically designed for neural processing, a dedicated AI accelerator/processing unit or an auxiliary processor among several processors associated with the computer 300, and the processor 310 may be a semiconductor based microprocessor (in the form of a microchip) or a microprocessor.

The memory 320 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 320 may incorporate electronic, magnetic, optical, and/or other types of storage media. Note that the memory 320 can have a distributed architecture, where various components are situated remote from one another, but can be accessed by the processor 310.

The software in the memory 320 may include one or more separate programs, each of which comprises an ordered listing of executable instructions for implementing logical functions. The software in the memory 320 includes a suitable operating system (O/S) 340, compiler 360, source code 350, and one or more applications 370 in accordance with exemplary embodiments. As illustrated, the application 370 comprises numerous functional components for implementing the features and operations of the exemplary embodiments. The application 370 of the computer 300 may represent various applications, computational units, logic, functional units, processes, operations, virtual entities, and/or modules in accordance with exemplary embodiments, but the application 370 is not meant to be a limitation.

The operating system 340 controls the execution of other computer programs, and provides scheduling, input-output control, file and data management, memory management, and communication control and related services. It is contemplated by the inventors that the application 370 for implementing exemplary embodiments may be applicable on all commercially available operating systems.

Application 370 may be a source program, executable program (object code), script, or any other entity comprising a set of instructions to be performed. When a source program, then the program is usually translated via a compiler (such as the compiler 360), assembler, interpreter, or the like, which may or may not be included within the memory 320, so as to operate properly in connection with the O/S 340. Furthermore, the application 370 can be written as an object oriented programming language, which has classes of data and methods, or a procedure programming language, which has routines, subroutines, and/or functions, for example but not limited to, C, C++, C#, Pascal, BASIC, API calls, HTML, XHTML, XML, ASP scripts, JavaScript, FORTRAN, COBOL, Perl, Java, ADA, .NET, and the like.

The I/O devices 330 may include input devices such as, for example but not limited to, a mouse, keyboard, scanner, microphone, camera, etc. Furthermore, the I/O devices 330 may also include output devices, for example but not limited to a printer, display, etc. Finally, the I/O devices 330 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 330 also include components for communicating over various networks, such as the Internet or intranet.

If the computer 300 is a PC, workstation, intelligent device or the like, the software in the memory 320 may further include a basic input output system (BIOS) (omitted for simplicity). The BIOS is a set of essential software routines that initialize and test hardware at startup, start the O/S 340, and support the transfer of data among the hardware devices. The BIOS is stored in some type of read-only-memory, such as ROM, PROM, EPROM, EEPROM or the like, so that the BIOS can be executed when the computer 300 is activated.

When the computer 300 is in operation, the processor 310 is configured to execute software stored within the memory 320, to communicate data to and from the memory 320, and to generally control operations of the computer 300 pursuant to the software. The application 370 and the O/S 340 are read, in whole or in part, by the processor 310, perhaps buffered within the processor 310, and then executed.

When the application 370 is implemented in software it should be noted that the application 370 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 370 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The proposed control method(s) of Fig. 5, and parts of the system(s) of Fig. 1 and Fig. 2, may be implemented in hardware or software, or a mixture of both (for example, as firmware running on a hardware device). To the extent that an embodiment is implemented partly or wholly in software, the functional steps illustrated in the process flow diagrams may be performed by suitably programmed physical computing devices, such as one or more central processing units (CPUs) or graphics processing units (GPUs). Each process - and its individual component steps as illustrated in the flow diagrams - may be performed by the same or different computing devices. According to embodiments, a computer-readable storage medium stores a computer program comprising computer program code configured to cause one or more physical computing devices to carry out a control method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, some of the blocks shown in the block diagrams may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

Variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flow diagrams and block diagrams in the Figures illustrate the architecture, functionality, and operation of possible implementations of systems, methods, and computer program products according to various embodiments of the present invention. In this regard, each block in the flow diagrams or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flow diagrams and combinations of blocks in the block diagrams and/or flow diagrams, can be implemented by special purpose hardware-based systems that perform the specified functions or acts or carry out combinations of special purpose hardware and computer instructions.

**Claims**

1.　A shaving system (100) comprising a shaving device (120), a sanitizing device (140), and a processing unit (160), wherein:

the shaving device comprises an internal member (122), an external member (124) covering the internal member and including hair entry openings (126) exposing parts of the internal member, and an actuator (130) configured to generate relative movement of the internal member and the external member, wherein the external member and the internal member are configured to cut hair passing through the hair entry openings during relative movement of the internal member and the external member;

the sanitizing device comprises a light source (142) configured to illuminate the external member and the exposed parts of the internal member with sanitizing light in a predefined sanitizing position of the shaving device relative to the sanitizing device; and

the processing unit (160) is configured to:

control (210) the light source to illuminate the external member and the exposed parts of the internal member with sanitizing light within a sanitizing time period; and

control (220) the actuator to generate relative movement of the internal member and the external member within the sanitizing time period such that different parts of the internal member are exposed to the sanitizing light via the hair entry openings during respective different parts of the sanitizing time period.

2. The shaving system of claim 1, further comprising a positioning arrangement (144) configured to position the shaving device (120) in the predefined sanitizing position relative to the sanitizing device (140).

3. The shaving system of claim 2, wherein the sanitizing device (140) comprises a casing configured to receive at least a shaving unit (128) of the shaving device, wherein the casing includes the positioning arrangement (144) and wherein the shaving unit includes the internal member (122) and the external member (124).

4. The shaving system of any of claims 1 to 3, wherein the processing unit (160) is part of the sanitizing device (140).

5. The shaving system of claim 4, wherein the processing unit (160) is configured to control the actuator (130) via data communication with a control unit (132) of the shaving device (120) enabled in the sanitizing position of the shaving device.

6. The shaving system of any of claims 1 to 5, wherein the processing unit (160) is configured to control the actuator (130) to continuously move the internal member (122) relative to the external member (124) throughout the sanitizing time period.

7. The shaving system of any of claims 1 to 5, wherein the processing unit (160) is configured to control the actuator (130) to move the internal member (122) relative to the external member (124) during one or more discrete parts of the sanitizing time period.

8. The shaving system of any of claims 1 to 7, wherein the processing unit (160) is configured to control the actuator (130) to move the internal member (122) relative to the external member (124) within the sanitizing time period at a predefined movement speed.

9. The shaving system of claim 8, wherein the processing unit (160) is further configured to control the actuator (130) to move the internal member (122) relative to the external member (124) at an operational movement speed for cutting hair, and wherein the predefined movement speed is less than the operational movement speed.

10. The shaving system of any of claims 1 to 9, wherein the processing unit (160) is configured to control the light source (142) to continuously illuminate the external member (124) and the exposed parts of the internal member (122) with the sanitizing light throughout the sanitizing time period.

11. The shaving system of any of claims 1 to 9, wherein the processing unit (160) is configured to control the light source (142) to illuminate the external member (124) and the exposed parts of the internal member (122) with sanitizing light at a predefined pulse frequency within the sanitizing time period.

12. The shaving system of claim 8 or 9, wherein the processing unit (160) is configured to:

control the light source (142) to illuminate the external member (124) and the exposed parts of the internal member (122) with the sanitizing light at a predefined pulse frequency within the sanitizing time period, and wherein the predefined movement speed and the predefined pulse frequency are adapted such that different parts of the internal member are exposed to the sanitizing light via the hair entry openings (126) during respective different parts of the sanitizing time period.

13. The shaving system of any of claims 1 to 12, wherein the processing unit (160) is configured to control the light source (142) and the actuator (130) to illuminate the external member (124) and the exposed parts of the internal member (122) with the sanitizing light while the actuator moves the internal member relative to the external member within the sanitizing time period.

14. The shaving system of any of claims 1 to 13, wherein the sanitizing light comprises ultraviolet light.

15. A sanitizing device (140) configured to sanitize a shaving device (120), wherein the shaving device comprises:

a shaving unit (128) having an internal member (122) and an external member (124) covering the internal member and including hair entry openings (126) exposing parts of the internal member; and

an actuator (130) configured to generate relative movement of the internal member and the external member, wherein the external member and the internal member are configured to cut hair passing through the hair entry openings during relative movement of the internal member and the external member,

wherein the sanitizing device comprises:

a casing configured to receive at least the shaving unit of the shaving device and including a positioning arrangement (144) configured to position the shaving device in a predefined sanitizing position relative to the sanitizing device;

a light source (142) configured to illuminate the external member and the exposed parts of the internal member with sanitizing light in the predefined sanitizing position of the shaving device; and

a processing unit (160) configured to, in the predefined sanitizing position of the shaving unit:

control (210) the light source to illuminate the external member and the exposed parts of the internal member with sanitizing light within a sanitizing time period; and

control (220) the actuator to generate relative movement of the internal member and the external member within the sanitizing time period such that different parts of the internal member are exposed to the sanitizing light via the hair entry openings during respective different parts of the sanitizing time period.

16. A method (200) for sanitizing a shaving device comprising an internal member, an external member covering the internal member and including hair entry openings exposing parts of the internal member, and an actuator configured to generate relative movement of the internal member and the external member, wherein the external member and the internal member are configured to cut hair passing through the hair entry openings during relative movement of the internal member and the external member, the method comprising:

controlling (210) a light source to illuminate the external member and the exposed parts of the internal member with sanitizing light in a predefined sanitizing position of the shaving device relative to the sanitizing device within a sanitizing time period; and

controlling (220) the actuator to generate relative movement of the internal member and the external member within the sanitizing time period such that different parts of the internal member are exposed to the sanitizing light via the hair entry openings during respective different parts of the sanitizing time period.

100

120

128    122    124

140

Actuator    130

Light Source    142

Positioning
Arrangement    144

Control
Unit    132

Processing
Unit    160

FIG. 1

**FIG. 2**

FIG. 3

124a

126

127

124b

126

127

FIG. 4

200

Control the light source — 210

Control the actuator — 220

FIG. 5

FIG. 6

Europäisches
Patentamt
European
Patent Office
Office européen
des brevets

# EUROPEAN SEARCH REPORT

Application Number

EP 24 15 9188

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 217 195 480 U (ZHEJIANG MENGLI ELECTRIC APPLIANCE TECH CO LTD) 16 August 2022 (2022-08-16) * paragraph: [0027-0030]; figure: 1, 2 * | 1-16 | INV. A61L2/08 A61L2/10 B26B21/00 |

TECHNICAL FIELDS
SEARCHED (IPC)

A61L
B26B

The present search report has been drawn up for all claims

| Place of search | Date of completion of the search | Examiner |
|---|---|---|
| Munich | 2 August 2024 | Nania, Manuela |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

                       

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 24 15 9188

This annex lists the patent family members relating to the patent documents cited in the above-mentioned European search report.
The members are as contained in the European Patent Office EDP file on
The European Patent Office is in no way liable for these particulars which are merely given for the purpose of information.

02-08-2024

| Patent document cited in search report | Publication date | Patent family member(s) | Publication date |
|---|---|---|---|
| CN 217195480 U | 16-08-2022 | NONE | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 20110126002 A
- KR 200350028 Y1
- CN 214963194 U